Europäisches Patentamt

European Patent Office

Office européen des brevets

(19)

(11) Veröffentlichungsnummer: **0 334 979**

**A1**

(12) **EUROPÄISCHE PATENTANMELDUNG**

(21) Anmeldenummer: 88105086.8

(22) Anmeldetag: 29.03.88

(51) Int. Cl.⁴: **A61N 5/06 , A47C 21/02 , A61G 7/06**

| | |
|---|---|
| Die Patentansprüche 17, 18, 19, 20, gelten durch Nichtzahlung der Anspruchsgebühren als verzichtet (Regel 31 (2) EPÜ). | (71) Anmelder: **Pürschel, Dieter**<br>**Martener Hellweg 32**<br>**D-4600 Dortmund 70(DE)** |
| (43) Veröffentlichungstag der Anmeldung:<br>**04.10.89 Patentblatt 89/40** | (72) Erfinder: **Pürschel, Dieter**<br>**Martener Hellweg 32**<br>**D-4600 Dortmund 70(DE)** |
| (84) Benannte Vertragsstaaten:<br>**DE FR GB IT** | (74) Vertreter: **Hemmerich, Friedrich Werner et al**<br>**Patentanwälte**<br>**HEMMERICH-MÜLLER-GROSSE-POLLMEIER--**<br>**MEY Eduard-Schloemann-Strasse 47**<br>**D-4000 Düsseldorf 1(DE)** |

(54) **Liegegestell für die Ganz-körperbestrahlung.**

(57) Ein Liegegestell für die Ganzkörperbestrahlung mit einer Körperauflageplatte 1 aus lichtdurchlässigem Werkstoff. Unterhalb dieser Körperauflageplatte 1 sind im Liegegestell Bräunungs-Strahleinrichtungen und Zusatzgeräte für diese angeordnet. Die Körperauflageplatte 1 wird von mehreren im Liegegestell 2 um horizontale Achsen schwenkbar gelagerte Kipprahmen getragen. Im Abstand e oberhalb der Auflageebene der Körperauflageplatte 1 befindet sich etwa parallel zu dieser ein mattenförmig gespanntes Aufliegetuch in Form eines Bandes 20, das über Umlenkrollen 21, 22 um die Körperauflageplatte 1 herum in eine Reinigungs -und Trockeneinrichtung 25, 26 und aus dieser heraus bewegbar ist.

FIG. 3

## Liegegestell für die Ganzkörper-Bestrahlung

Die Erfindung bezieht sich auf ein Liegegestell für die Ganzkörperbestrahlung mit einer Körperauflageplatte aus lichtdurchlässigem Werkstoff und unterhalb dieser Platte im Liegegestell angeordnete Bräunungs-Strahleinrichtungen und Zusatzgeräte für diese.

Bekannte Liegegestelle dieser Art weisen durchweg ebene oder auch quer zur Körperauflagelängsrichtung konkav gewölbte Körperauflageplatten auf, die eine Liegebank oder einen Liegetisch darstellen, der ggfs. von Hand oder motorisch in seiner Höhe verstellt werden kann. Für den Kopf der zu bestrahlenden Person sind Kopfauflagen vorgesehen.

Der Nachteil dieser Liegegestelle besteht insb. bei längerer Bestrahlungsdauer in der Unbequemlichkeit der Liegestellung für die Person, die nicht deren natürlicher Liegestellung entspricht; sie führt dabei leicht zu Ermüdungen und auch zu Muskelverspannungen.

Der Erfindung liegt die Aufgabe zugrunde, die bekannten Liegegestelle dieser Art so zu verbessern, daß die Liegestellung der zu bestrahlenden Person deren individuellen Wünschen weitgehend angepaßt werden kann, ohne daß dabei die Bestrahlungsintensität und -qualität vermindert wird.

Diese Aufgabe wird durch einen oder mehrere, die Körperauflageplatte tragende, im Liegegestell um horizontale Achsen schwenkbar gelagerte Kipprahmen gelöst. Wie die Erfindung weiter vorsieht, kann dabei ein Kipprahmen aus einem Stück oder aus zwei oder mehr quer zur Körperauflagelängsrichtung schwenkbar aneinandergelenkten Teilabschnitten und die Körperauflageplatte ggfs. aus elastischen Werkstoffen bestehen. Die Kipprahmen bzw. die Achsen der Kipprahmen werden vorteilhaft mit im Liegegestell angeordneten Motorantrieben verbunden und im Liegegestell kann seitlich neben der Körperauflageplatte am Kipprahmen eine Betätigungsschaltergruppe für die Strahleinrichtungen und die Steuereinrichtungen für die Motorantriebe vorgesehen werden. Zwischen den oder dem Kipprahmen und dem Liegegestell werden vorteilhaft Balgen aus flexiblen Werkstoffen, z.B. Faltenbalge, angeordnet und die Körperauflageplatte kann die Form eines Liegesessels aufweisen.

Bei Liegegestellen, die mit einer mit veränderund festlegbarem Abstand über der Körperauflageplatte angeordneten, deren Größe angepaßten gegen die Körperauflageplatte gerichtete Strahleinrichtungen und Zusatzgeräten für diese aufnehmenden Bestrahlungskappe ausgestattet sind, die an einem mit dem Liegegestell verbundenen Tragständer angeordnet ist, können erfindungsgemäß so ausgebildet werden, daß die Bestrahlungskappe um eine horizontale Achse schwenkbar in diesem Tragständer gelagert ist und gegenüber der Körperauflageplatte abstandsverstellbar ist. Die Strahlungskappe kann dabei ebenfalls aus zwei oder mehr Teilabschnitten bestehen, die um quer zur Körperauflagerichtung liegende Achsen schwenkbar sind. Die in der Bestrahlungskappe angeordneten Strahleinrichtungen werden erfindungsgemäß gegenüber den winklig gegeneinander verlaufenden Teilflächen der Körperauflageplatte mit gleichbleibendem Abstand angeordnet, ggfs. sind sie dabei gegenüber der Körperauflageplatte abstandsverstellbar.

Diese Ausbildung des Liegegestells erlaubt es, die Körperauflageplatte der von der zu bestrahlenden Person bevorzugten Liegestellung durch Verschwenkung der Kipprahmen gegeneinander anzupassen und ggfs. auch noch in ihrer Lage zueinander zu verändern, wenn die Person ihre Liegestellung bereits eingenommen hat. Die Person kann diese Anpassung mit Hilfe der im Liegegestell angeordneten Betätigungsschalter selbst vornehmen, wenn sie dies wünscht. Wenn die Körperauflage die Form eines Liegesessels aufweist, kann dieser als Ganzes in eine gewünschte bequeme Kippstellung geschwenkt werden. Es besteht auch die Möglichkeit für das Liegegestell über die Betätigungsschalter eine Einstiegsstellung vorzuprogrammieren, die bei mit dem Fußteil der Körperauflageplatte nach unten geklappter Stellung das Ein- und später das Aussteigen aus dem Liegegestell, insb. bei behinderten Personen, erheblich erleichtert.

Die Erfindung erlaubt es weiter, im Abstand oberhalb der Auflageebene der Körperauflageplatte, etwa parallel zu dieser, ein mattenförmig gespanntes Aufliegetuch anzuordnen. Dieses Aufliegetuch nimmt die Person wie eine über die Körperauflageplatte gespannte Hängematte auf und verhindert, daß insb. deren Rücken, Schulter- und Steißbeinpartien, die sonst unmittelbar auf der Körperauflageplatte aufliegen, nach der Bestrahlung Druckmarkierungen zeigt. Wenn das Aufliegetuch, wie die Erfindung vorsieht, in Form eines Bandes über Umlenkrollen um die Körperauflageplatte herum in eine Reinigungs -und Trockeneinrichtung und aus dieser heraus bewegbar ist, ergibt sich der Vorteil der schnellen Bereitstellung eines hygienisch einwandfreien Aufliegetuches für die folgende Person.

Weitere Merkmale der Erfindung sind Gegenstand von Unteransprüchen. Die Erfindung wird nachstehend anhand der in der Zeichnung dargestellten Ausführungsbeispiele näher erläutert. In der Zeichnung zeigen

Fig. 1 das Liegegestell von der Seite gesehen mit der Abdeckklappe,

Fig. 2 das Liegegestell nach Fig. 1 seitlich von oben gesehen in perspektivischer Darstellung, teilweise geschnitten und

Fig. 3 eine andere Ausbildungsform des Liegegestells ebenfalls von der Seite gesehen in schematischer Darstellung.

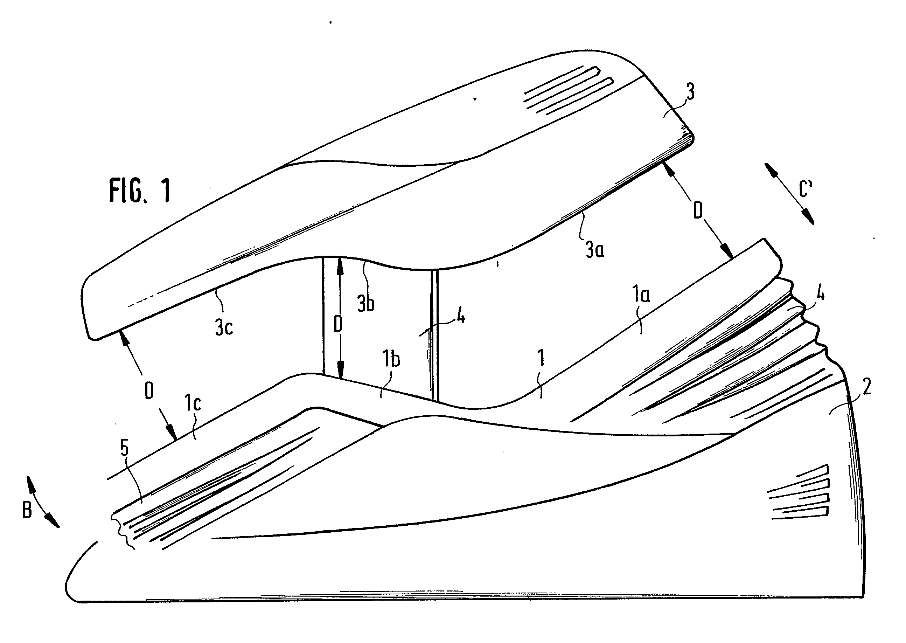

Wie aus Fig. 1 zu ersehen, ist das hier in Form eines Liegesessels mit Körperauflageplatte 1 ausgebildete Liegegestell 2 mit einer Abdeckhaube 3 ausgestattet, die auf einem Traggestell 4 befestigt ist. Die Auflageplatte besteht aus drei Teilabschnitten 1a, 1b und 1c, deren Auflageflächen stumpfwinklig zueinander verlaufend einen Liegesessel für die -nicht dargestellte - zu bestrahlende Person bilden. Die diesen Abschnitten 1a, 1b, 1c der Körperauflagefläche 1 zugewandten Abschnitte 3a, 3b und 3c der Abdeckkappe 3 weisen einen gleichbleibenden Abstand D zu den entsprechenden Teilabschnitten 1a, 1b und 1c auf. Zwischen dem die Körperauflageplatte 1 tragenden, in dieser Figur nicht dargestellten Rahmen und dem Liegegestell 2 sind Faltenbalge 4 und 5 vorgesehen, die dem durch die Doppelpfeile B und C angedeuteten Schwenkbewegungen der Körperauflageplatte 1 im Liegegestell 2 folgen können.

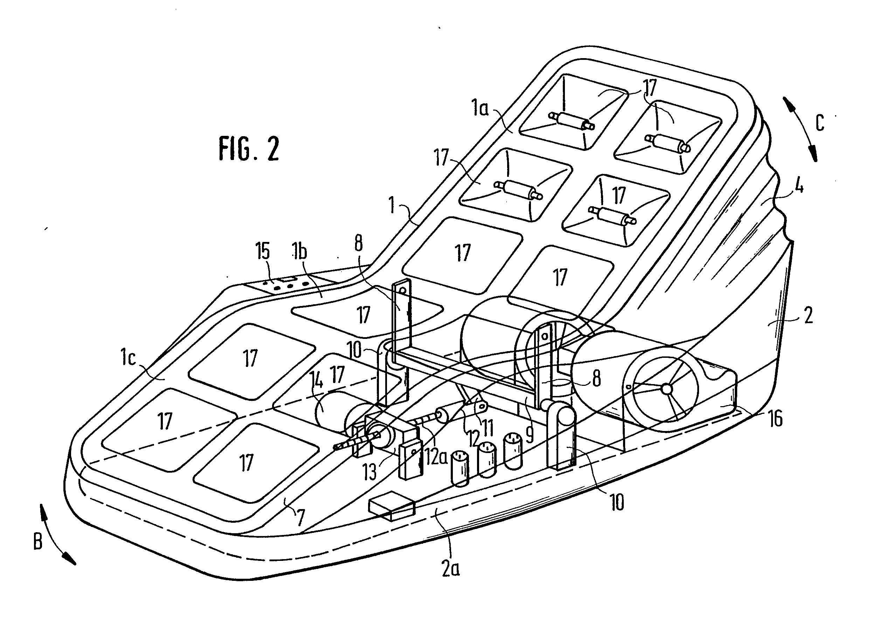

Aus Fig. 2 geht hervor, daß im Liegegestell 2 der die Körperauflageplatte 1 tragende Rahmen 7 von Tragflanschen 8 gehalten wird, die auf einer Achse 9 sitzen, die in Lagerböcken 10 lagert, die fest mit dem Tragrahmen 2a des Liegegestells verbunden ist. Die Achse 9 ist über einen Hebel 11 mit einem Schwenkspindelträger 12 verbunden, dessen Spindel 12a über einen Getriebeblock 13 von einem Motor 14 angetrieben wird. Dieser Motor 14 wird über nicht dargestellte Steuerelemente von der Betätigungsschaltertafel 15 aus gesteuert, die seitlich neben dem Teilabschnitt 1b der Körperauflagefläche 1 angeordnet ist. Im rückwärtigen Teil des Liegegestells 2 sind Lüfter 16 und andere Steuerelemente für den Betrieb der unterhalb der Körperauflageplatte 1 in dem Rahmen 7 angeordnete Bräunungs-Strahleinrichtungen 17 angeordnet.

Die Körperauflageplatte kann hier aus der gezeichneten Stellung bei entsprechender Betätigung der Schalter 15 in Richtung der beiden Doppelpfeile B und C geschwenkt und in jeder gewünschten Stellung festgehalten werden, wobei die Abdeckkappe 3 (vgl. Fig. 1) diesen Bewegungen auf nicht dargestellte Weise folgend angetrieben wird.

Wie bereits anfangs geschildert, können zwischen den Teilabschnitten 1a, 1b, 1c der Körperauflageplatte 1 bei entsprechender Gestaltung von diese Teilabschnitte tragenden, gelenkig miteinander verbundenen Rahmenteilen des hier einteilig ausgebildeten Rahmens 7 und Anordnung entsprechender Zusatzantriebe auch die Winkel der einzelnen Teilabschnitte der Körperauflageplatte gegeneinander verschwenkt und verändert werden, wobei die entsprechende Abstands- und Lageverstellung der Bräunungs-Strahleinrichtungen in der Abdeckkappe 3 mit Hilfe von in dieser Abdeckkappe angeordneten Stellmotoren bewirkt. wird.

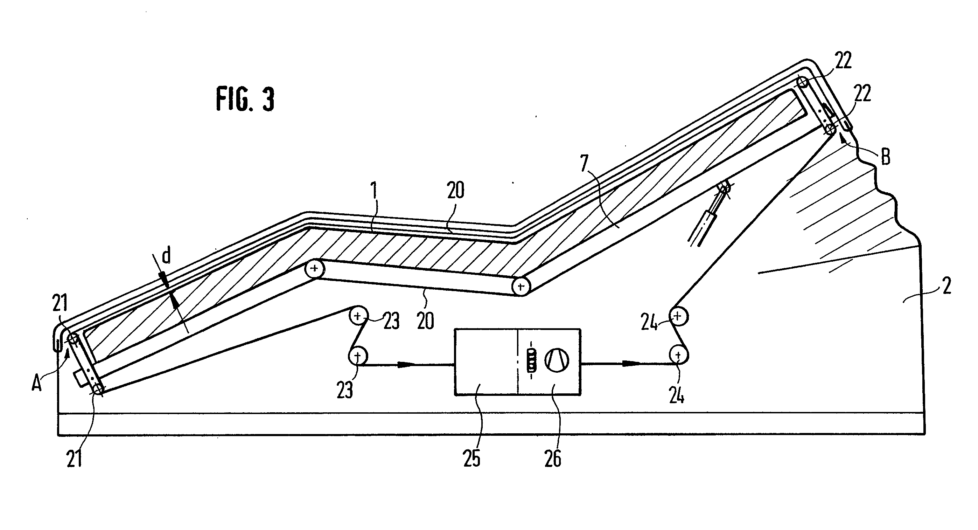

Beim Ausführungsbeispiel nach Fig. 3 ist im Abstand d über der Körperauflageplatte 1 etwa parallel zu deren Auflageebene ein Aufliegetuch in Form eines - hier - endlosen Bandes 20 um Umführungsrollenpaare 21 und 22 geführt. Dieses Band 20 läßt sich über Spannrollenpaare 23 und 24 spannen, die unterhalb des Rahmens 7 im Liegegestell 2 fest angeordnet sind. Zwischen den Spannrollenpaaren 23, 24 sind ebenfalls im Liegegestell 2 die Reinigungseinrichtung 25 und die dieser nachgeordnete Trockeneinrichtung 26 angeordnet. In den Abstandsraum zwischen Band 20 und Körperauflageplatte 1 kann mit bekannten Mitteln, wie Lüftern und Düsen, in Richtung der angedeuteten Pfeile A und B Kühlluft eingeblasen und ggfs. auch mit entsprechenden Saugeinrichtungen wieder abgezogen werden. Es besteht auch die - nicht dargestellte -Möglichkeit, solche Einrichtungen seitlich des Bandes 20 anzuordnen.

Das Band 20, das bspw. aus einem Acrylgespinst bestehen kann, wird zweckmäßig an beiden Seitenrändern mit Perforationslöchern ausgestattet, in die entsprechend ausgebildete Noppen auf den Umlenkrollenpaaren 21, 22 und ggfs. auf den Spannrollenpaaren 23, 24 eingreifen. Die Perforationen können von -nicht dargestellten - übergreifenden Kulissenleisten, und es besteht auch die Möglichkeit, die Seitenränder des Bandes 20 mit im Liegegestell 2 parallel zur Bandbewegungsrichtung verschieb- und in diesem festlegbaren Ankerelementen kuppelbar zu gestalten.

Das Band 20 kann nach jeder Benutzung von der Position über der Körperauflageplatte 1 umlaufend abgezogen, gereinigt und getrocknet wieder in Bereitschaftsposition gebracht werden. Es ist dabei auch möglich, in den Bandumlauf einen Speicherabschnitt einzubringen.

## Ansprüche

1. Liegegestell für die Ganzkörper-Bestrahlung mit einer Körperauflageplatte aus lichtdurchlässigem Werkstoff und unterhalb dieser Platte im Liegegestell angeordnete Bräunungs-Strahleinrichtungen und Zusatzgeräte für diese,
**gekennzeichnet durch**
einen oder mehrere, die Körperauflageplatte (1) tragenden, im Liegegestell (2) um horizontale Achsen (9) schwenkbar gelagerte Kipprahmen (7).

2. Liegegestell nach Anspruch 1,

**dadurch gekennzeichnet,**

daß ein Kipprahmen aus einem Stück (7) oder aus zwei oder mehr quer zur Körperauflagerichtung schwenkbar aneinander angelenkten Teilabschnitten besteht.

3. Liegegestell nach den Ansprüchen 1 und/oder 2,

**dadurch gekennzeichnet,**

daß die Körperauflageplatte (1) die Form eines Liegesessels aufweist.

4. Liegegestell nach einem oder mehreren der Ansprüche 1 bis 3,

**dadurch gekennzeichnet,**

daß die Körperauflageplatte (1) aus einem elastischen Werkstoff besteht.

5. Liegegestell nach einem oder mehreren der Ansprüche 1 bis 4,

**dadurch gekennzeichnet,**

daß die Kipprahmen (7) bzw. die Kipprahmenachse (9) mit im Liegegestell (2) angeordneten Motorantrieben (13, 14) verbunden sind.

6. Liegegestell nach Anspruch 5,

**gekennzeichnet durch**

im Liegegestell angeordnete, seitlich neben der Körperauflageplatte (2) am Kipprahmen (7) oder dem Liegegestell angeordnete Betätigungsschaltergruppen (15) für die Steuereinrichtungen und Motorantriebe.

7. Liegegestell nach einem oder mehreren der Ansprüche 1 bis 6,

**dadurch gekennzeichnet,**

daß zwischen dem Kipprahmen (7) und dem Liegegestell (2) an sich bekannte Balgen (4, 5) aus flexiblem Werkstoff angeordnet sind.

8. Liegegestell nach Anspruch 7,

**dadurch gekennzeichnet,**

daß die Balgen (4, 5) als Faltenbalge ausgebildet sind.

9. Liegegestell nach einem oder mehreren der Ansprüche 1 bis 8,

mit einer, mit veränder- und festlegbarem Abstand über der Körperauflageplatte angeordneten, deren Größe angepaßten, gegen die Körperauflageplatte gerichtete Strahleinrichtungen und Zusatzgeräte für diese aufnehmenden, an einem mit dem Liegegestell verbundenen Tragständer angeordneten Bestrahlungskappe,

**dadurch gekennzeichnet,**

daß die Bestrahlungskappe (3) um eine horizontale Achse schwenkbar in dem Tragständer (4) gelagert ist.

10. Liegegestell nach Anspruch 9,

**dadurch gekennzeichnet,**

daß die Bestrahlungskappe (3) gegenüber der Körperauflageplatte (1) abstandsverstellbar ist.

11. Liegegestell nach den Ansprüchen 9 und/oder 10,

**dadurch gekennzeichnet,**

daß die Bestrahlungskappe (3) aus zwei oder mehr Teilabschnitten besteht, die um quer zur Körperauflagerichtung liegende Achsen schwenkbar sind.

12. Liegegestell nach einem oder mehreren der Ansprüche 9 bis 11,

**dadurch gekennzeichnet,**

daß die in der Bestrahlungskappe (3) angeordneten Strahleinrichtungen gegenüber den winklig zueinander verlaufenden Teilflächen der Körperauflageplatte (1) mit gleichbleibendem Abstand angeordnet sind.

13. Liegegestell nach Anspruch 12,

**dadurch gekennzeichnet,**

daß die Strahleinrichtungen in der Bestrahlungskappe (3) gegenüber der Körperauflageplatte (1) abstandsverstellbar sind.

14. Liegegestell nach einem oder mehreren der Ansprüche 1 bis 13,

**gekennzeichnet durch,**

ein im Abstand (d) oberhalb der Auflageebene der Körperauflageplatte (1) etwa parallel zu dieser mattenförmig gespanntes Aufliegetuch.

15. Liegegestell nach Anspruch 14,

**dadurch gekennzeichnet,**

daß das Auflagetuch in Form eines Bandes (20) über Umlenkrollen (21, 22) um die Körperauflageplatte (1) herum in eine Reinigungs- und Trockeneinrichtung (25, 26) und aus dieser herausbewegbar ist.

16. Liegegestell nach den Ansprüchen 14 und/oder 15,

**dadurch gekennzeichnet,**

daß die Bewegung des Bandes (20) eine Reversierbewegung ist.

17. Liegegestell nach einem oder mehreren der Ansprüche 14 bis 16,

**dadurch gekennzeichnet,**

daß die Seitenränder des Bandes (20) mit im Liegegestell (2) parallel zur Bandbewegungsrichtung verschieb- und in diesem festlegbaren Ankerelementen kuppelbar sind.

18. Liegegestell nach Anspruch 17,

**dadurch gekennzeichnet,**

daß die Seitenränder des Bandes (20) Perforationen für in diese eingreifende radiale Noppen der Umlenkrollen (21, 22) bzw. der Spannrollen (23, 24) und/oder der Ankerelemente aufweisen.

19. Liegegestell nach einem oder mehreren der Ansprüche 14 bis 18,

**dadurch gekennzeichnet,**

daß die Perforationen in übergreifenden Kulissenleisten des Liegegestells (2) geführt sind.

20. Liegegestell nach einem oder mehreren der Ansprüche 14 bis 19,

**gekennzeichnet durch,**

den Raum zwischen Aufliegetuch bzw. Band (20) und Körperauflageplatte (1) mit kühlluftbeaufschlagende Blasdüsen bzw. diese absaugende Saugstutzen.

1 5. Mai. 1988

FIG. 1

FIG. 2

FIG. 3

EP 0 334 979 A1

| | Europäisches Patentamt | EUROPÄISCHER RECHERCHENBERICHT | Nummer der Anmeldung |
|---|---|---|---|
| | | | EP 88 10 5086 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | DE-A-3 632 927 (PÜRSCHEL)<br>* Insgesamt *<br>--- | 1-13 | A 61 N 5/06<br>A 47 C 21/02<br>A 61 G 7/06 |
| X | DE-A-3 630 060 (PHILIPS)<br>* Insgesamt *<br>--- | 1-3,9-13 | |
| A | US-A-4 494 259 (MILLER)<br>* Spalte 2, Zeile 54 - Spalte 3, Zeile 33; Spalte 4, Zeile 41 - Spalte 5, Zeile 41 *<br>--- | 1-8 | |
| A | US-A-4 003 704 (ZUROLO)<br>* Insgesamt *<br>----- | 14-16 | |
| | | | **RECHERCHIERTE SACHGEBIETE (Int. Cl.4)**<br><br>A 61 N<br>A 47 C<br>A 61 G |

~~Der vorliegende Recherchenbericht wurde für alle Patentansprüche erstellt~~

| Recherchenort | Abschlußdatum der Recherche | Prüfer |
|---|---|---|
| DEN HAAG | 15-11-1988 | LEMERCIER D.L.L. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

Europäisches
Patentamt

## ☒ GEBÜHRENPFLICHTIGE PATENTANSPRÜCHE

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

☒ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt für die Anspruchsgebühren entrichtet wurden, 11-16

nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen, nämlich:

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche: